(19) Europäisches Patentamt
European Patent Office
Office européen des brevets

(11) EP 0 998 904 A1

(12) DEMANDE DE BREVET EUROPEEN

(43) Date de publication:
10.05.2000 Bulletin 2000/19

(51) Int Cl.[7]: **A61K 7/02**, A61K 7/027

(21) Numéro de dépôt: **99402437.0**

(22) Date de dépôt: **05.10.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **03.11.1998 FR 9813806**

(71) Demandeur: **L'OREAL**
**75008 Paris (FR)**

(72) Inventeurs:
- **Lemann, Patricia**
  **94000 Créteil (FR)**
- **Auguste, Frédéric**
  **94550 Chevilly-Larue (FR)**
- **Cantin, Hervé**
  **91420 Morangis (FR)**

(74) Mandataire: **Brédeville, Odile Marie**
**L'Oreal,**
**D.P.I.,**
**6, rue Bertrand Sincholle**
**92585 Clichy Cédex (FR)**

# (54) Procédé de maquillage sans transfert, compositions et kit pour la mise en oeuvre d'un tel procédé

(57) La présente invention est relative à un procédé pour améliorer les propriétés 'sans transfert' et la tenue d'une composition A comprenant au moins une phase grasse comprenant une partie non volatile de paramètres de solubilité de Hansen $\delta^a_d$, $\delta^a_p$, $\delta^a_h$, comprenant les étapes suivantes :

- 1°) dans un premier temps, on applique sur la peau et/ou les muqueuses et/ou les semi-muqueuses une base cosmétique B comprenant au moins une phase grasse dont la partie non volatile a des paramètres de solubilité de Hansen $\delta^b_d$, $\delta^b_p$, $\delta^b_h$, B étant choisie de telle façon que la distance séparant A de B dans l'espace des paramètres de solubilité de Hansen soit supérieure ou égale à 3 $J^{1/2}cm^{-3/2}$,
- 2°) dans un deuxième temps, on applique sur la base B la composition A. Elle porte également sur des compositions permettant la mise en oeuvre de ce procédé ainsi que sur un kit comprenant ces compositions.

EP 0 998 904 A1

## Description

La présente invention a pour objet un procédé pour améliorer le 'sans transfert' et la tenue d'une composition, en particulier de maquillage, telle que par exemple les fonds de teint, les poudres, les rouges à lèvres.

Les compositions cosmétiques, notamment de maquillage telles que les rouges à lèvres, les fonds de teint, les produits de maquillage du corps, les anticernes, les fards à paupières ou les poudres, comprennent généralement des corps gras tels que des huiles et/ou des cires, et une phase particulaire généralement composée de charges et de pigments. Elles peuvent ainsi se présenter sous la forme d'un gel anhydre, sous forme de stick ou bâton ou sous forme de pâte souple, comme par exemple certains fonds de teint, fards à paupières ou rouges à lèvres. Elles peuvent également se présenter sous la forme d'une poudre, qui peut être par exemple libre, compactée ou pressée. Les compositions de maquillage peuvent également comprendre de l'eau ou une phase hydrophile, et se présenter alors notamment sous forme d'émulsion huile-dans-eau, eau-dans-huile, émulsion multiple, notamment lorsqu'il s'agit d'un fond de teint, de crème teintée, de crème de soin ou d'un produit solaire.

Ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau.

En se déposant, ladite composition laisse une trace sur ledit support. Il s'en suit donc une persistance médiocre de la composition sur la peau ou les muqueuses, d'où la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières.

Tous ces phénomènes engendrent un effet inesthétique que l'on souhaite bien évidemment éviter.

Depuis plusieurs années, de nombreux cosméticiens se sont intéressés aux compositions cosmétiques, notamment de rouges à lèvres ou de fonds de teint 'sans transfert'. Ainsi, les produits de maquillage dits 'sans transfert' actuels sont souvent obtenus par formation d'un film de polymères par évaporation d'un solvant. Or, la formulation de molécules complexes tels que les polymères dans des produits de maquillage n'est pas aisée et n'est pas toujours souhaitable en ce qui concerne le toucher, la texture du produit et son confort.

Il est également connu que la présence d'huiles volatiles au sein de ces produits de maquillage améliore leurs propriétés 'sans transfert'. On peut associer à ces huiles volatiles des composés siliconés.

Toutefois, si on sait maintenant que l'association d'huiles volatiles avec certains composés siliconés permet d'obtenir un résultat 'sans transfert' satisfaisant, elle présente néanmoins l'inconvénient de conduire, après évaporation des volatils, à un film dont le confort n'est pas optimal, notamment parce qu'il n'est pas possible d'ajouter d'huiles autres que siliconées dans ces compositions tout en conservant une qualité de 'sans transfert' correcte. En effet, les huiles hydrocarbonées, qui sont connues pour apporter notamment du confort à une composition cosmétique, ont comme inconvénient d'augmenter le transfert d'une telle composition.

Il en résulte que les produits de maquillage actuels dits 'sans transfert' sont souvent peu confortables après séchage et présentent souvent un film sec non hydratant.

Il subsiste donc le besoin de produits, notamment de maquillage, qui transfèrent peu ou pas du tout (c'est-à-dire de produits 'sans transfert') tout en possédant de bonnes propriétés cosmétiques, notamment des propriétés de glissant à l'application, ainsi que des qualités de douceur, d'hydratation et de confort après maquillage.

Or, la Demanderesse a trouvé de manière surprenante qu'en combinant de façon particulière deux compositions cosmétiques, chacune de ces deux compositions étant connue en soi, il était possible de réaliser un produit de maquillage 'sans transfert' comprenant des huiles non volatiles.

On réalise ainsi des produits de maquillage qui, non seulement sont 'sans transfert' mais qui possèdent également de bonnes propriétés cosmétiques, en particulier de confort et d'hydratation.

L'invention a donc pour objet un procédé pour améliorer les propriétés 'sans transfert' et la tenue d'une composition A comprenant au moins une phase grasse dont la partie non volatile a des paramètres de solubilité de Hansen $\delta^a_d$, $\delta^a_p$, $\delta^a_h$, comprenant les étapes suivantes :

- 1°) dans une première étape, on applique sur la peau et/ou les muqueuses et/ou les semi-muqueuses une base cosmétique B comprenant au moins une phase grasse dont la partie non volatile a des paramètres de solubilité de Hansen $\delta^b_d$, $\delta^b_p$, $\delta^b_h$, B étant choisie de telle façon que la distance d(A-B) séparant A de B dans l'espace des paramètres de solubilité de Hansen vérifie la relation suivante :

$$d(A\text{-}B) \geq 3\ J^{1/2}cm^{-3/2},$$

dans laquelle :

$$d(A\text{-}B) = \sqrt{4(\delta^a_d - \delta^b_d)^2 + (\delta^a_p - \delta^b_p)^2 + (\delta^a_h - \delta^b_h)^2}$$

- 2°) dans une deuxième étape, on applique sur la base B la composition A.

La présente invention a encore pour objet un procédé de maquillage, en particulier de la peau et/ou des muqueuses et/ou des semi-muqueuses, comprenant les étapes suivantes :

- 1°) dans une première étape, on applique sur la peau et/ou les muqueuses et/ou les semi-muqueuses une base cosmétique B comprenant au moins une phase grasse dont la partie non volatile a des paramètres de solubilité de Hansen $\delta^b_d$, $\delta^b_p$, $\delta^b_h$,

- 2°) dans une deuxième étape, on applique sur la base B une composition A comprenant au moins une phase grasse dont la partie non volatile a des paramètres de solubilité de Hansen $\delta^a_d$, $\delta^a_p$, $\delta^a_h$,

A et B étant choisies de telle façon que la distance d(A-B) séparant A de B dans l'espace des paramètres de solubilité de Hansen vérifie la relation suivante :

$$d(A\text{-}B) \geq 3\ J^{1/2}cm^{-3/2},$$

dans laquelle :

$$d(A\text{-}B) = \sqrt{4(\delta^a_d - \delta^b_d)^2 + (\delta^a_p - \delta^b_p)^2 + (\delta^a_h - \delta^b_h)^2}$$

La composition A appliquée sur la base cosmétique B selon le procédé ci-dessus présente l'avantage de transférer très peu, voire pas du tout, tout en conservant ses propriétés de confort et d'hydratation. Elle peut également ne pas comprendre de polymère du tout : la composition est alors particulièrement confortable.

La composition A présente également une très bonne tenue. Elle est très agréable au toucher et s'applique facilement.

La présente invention a également pour objet une composition A, en particulier cosmétique, notamment de maquillage, comprenant une phase grasse, caractérisée par le fait que les paramètres de solubilité de Hansen $\delta^a_d$, $\delta^a_p$, $\delta^a_h$ de la partie non volatile de ladite phase grasse sont choisis de telle façon que la distance d(A-B) séparant, dans l'espace des paramètres de solubilité de Hansen, A d'une composition B telle que définie ci-dessus vérifie la relation suivante :

$$d(A\text{-}B) \geq 3\ J^{1/2}cm^{-3/2},$$

dans laquelle :

$$d(A\text{-}B) = \sqrt{4(\delta^a_d - \delta^b_d)^2 + (\delta^a_p - \delta^b_p)^2 + (\delta^a_h - \delta^b_h)^2}$$

et où $\delta^b_d$, $\delta^b_p$, $\delta^b_h$ sont les paramètres de solubilité de Hansen de la partie non volatile de la phase grasse de B.

La présente invention a encore pour objet une base cosmétique B, notamment de soin, comprenant une phase grasse, caractérisée par le fait que les paramètres de solubilité de Hansen $\delta^b_d$, $\delta^b_p$, $\delta^b_h$ de la partie non volatile de ladite phase grasse sont choisis de telle façon que la distance d(A-B) séparant, dans l'espace des paramètres de solubilité de Hansen, B d'une composition A telle que définie ci-dessus vérifie la relation suivante :

$$d(A\text{-}B) \geq 3\ J^{1/2}cm^{-3/2},$$

dans laquelle :

$$d(A\text{-}B) = \sqrt{4(\delta^a_d - \delta^b_d)^2 + (\delta^a_p - \delta^b_p)^2 + (\delta^a_h - \delta^b_h)^2}$$

et où $\delta^a_d$, $\delta^a_p$, $\delta^a_h$ sont les paramètres de solubilité de Hansen de la partie non volatile de la phase grasse de A.

Un autre objet de l'invention porte sur l'utilisation d'une composition A telle que définie ci-dessus, en particulier de maquillage, pour la mise en oeuvre des procédés ci-dessus.

Encore un autre objet de l'invention porte sur l'utilisation d'une base cosmétique B telle que définie ci-dessus, en particulier de soin, pour la mise en oeuvre des procédés ci-dessus.

La présente invention a encore pour objet un kit comprenant une composition A et une base cosmétique B telles que définies ci-dessus.

Un autre objet de l'invention réside dans un produit de maquillage sans transfert bicouche constitué d'une première couche, en contact avec la peau, les muqueuses et/ou les semi-muqueuses, constituée de la base cosmétique B et d'une deuxième couche, constituée de la composition A, apposée sur la première couche.

Les compositions selon l'invention sont facilement applicables sur la peau et permettent entre autre d'obtenir des produits de maquillage ayant une texture peu collante, qui restent confortables à porter tout au long de la journée.

De plus, leurs propriétés cosmétiques sont très intéressantes : elles procurent une grande douceur en final et un maquillage unifiant et confortable.

Les compositions selon l'invention trouvent notamment une application particulièrement intéressante dans le domaine du soin et/ou du maquillage de la peau, des muqueuses, des semi-muqueuses, et des phanères. On entend notamment par muqueuse, la partie interne de la paupière inférieure; par semi-muqueuses, on entend plus particulièrement les lèvres du visage; par phanères, on entend les cils, sourcils, cheveux et ongles. Ainsi, l'invention trouve une application toute particulière dans le domaine des produits de soin et/ou de maquillage des lèvres du visage et de la peau, tels que les fonds de teint, les anticernes, les fards à paupières, les poudres, les produits de maquillage du corps, les rouges à lèvres, les autobronzants ou les produits solaires.

D'autres caractéristiques, aspects et avantages de la présente invention apparaîtront à la lecture de la description détaillée qui va suivre.

La composition A selon l'invention comprend nécessairement une phase grasse comprenant une partie non volatile.

Cette partie non volatile peut comprendre tous corps gras non volatils habituellement utilisés en cosmétique tels que les huiles non volatiles, les cires, les gommes, les résines et/ou les corps gras pâteux d'origine animale, végétale, minérale ou synthétique, ainsi que leurs mélanges.

Par huile non volatile, on entend un corps gras liquide à température ambiante et qui ne s'évapore pas à cette même température.

Parmi les huiles non volatiles, on peut citer :

- les polyalkyl($C_1$-$C_{20}$) siloxanes et notamment ceux à groupements terminaux triméthylsilyle, de préférence ceux dont la viscosité est inférieure à 0,06 $m^2$/s parmi lesquels on peut citer les polydiméthylsiloxanes linéaires et les alkylméthylpolysiloxanes tels que la cétyldiméthicone (nom CTFA),

- les silicones modifiées par des groupements aliphatiques et/ou aromatiques, éventuellement fluorés, ou par des groupements fonctionnels tels que des groupements hydroxyles, thiols et/ou amines.

- les huiles de silicone phénylées, notamment celles de formule :

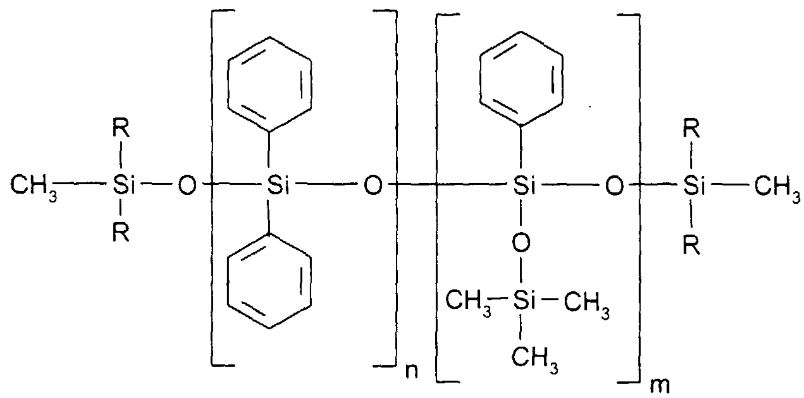

(I)

dans laquelle R est un radical alkyle en $C_1$-$C_{30}$, un radical aryle ou un radical aralkyle, n est un nombre entier compris entre 0 et 100, et m est un nombre entier compris entre 0 et 100, sous réserve que la somme est comprise entre 1 et 100;

- les huiles d'origine animale, végétale ou minérale, telles que l'huile de paraffine, de vaseline, le perhydrosqualène, la lanoline, l'huile d'abricot, l'huile de germes de blé, d'amande douce, de calophyllum, de sésame, de macadamia, de pépins de raisin, de colza, de coprah, d'arachide, de tournesol, de palme, de ricin, d'avocat, de jojoba, d'olive ou de germes de céréales, la fraction liquide du beurre de karité; des esters d'acides gras et de polyol, en particulier les triglycérides liquides, le myristate d'isopropyle, l'huile de miglyol, le néopentanoate d'isostéaryle, le palmitate d'éthyl-2-hexyle, l'huile de ricin, l'acétyl-citrate de tri-butyle; des alcools, en particulier l'octyl-2-dodecanol ; des acétylglycérides ; des octanoates, décanoates ou ricinoléates d'alcools ou de polyalcools ; des triglycérides d'acides gras; des glycérides, les huiles fluorées et perfluorées ;

- les composés amidés, en particulier ceux décrits dans la demande PCT/FR98/01077 comme le N-néopentanoyl-2-octyl-dodécylamine, le N-néopentanoyl-2-butyl-octylamine, le N-(3,5,5-triméthyl-hexanoyl)-2-octyldodécylamine, le N-(3,5,5-triméthyl-hexanoyl)-2-butyl-octylamine,

- leurs mélanges.

De préférence, la partie non volatile de la phase grasse de la composition A comprend une ou plusieurs huiles choisies parmi l'huile de sésame, la lanoline, le myristate d'isopropyle, l'huile de mygliol, le néopentanoate d'isostéaryle, le palmitate d'éthyl-2-hexyle, l'huile de ricin, l'acétyl-citrate de tri-butyle, l'octyl-2-dodecanol, le N-pentanoyl-2-octyl dodecylamine.

De préférence, les huiles non volatiles sont présentes dans la composition A selon l'invention à une teneur pouvant aller de 1 à 85%, de préférence encore de 5 à 50%, en poids, par rapport au poids total de la composition A.

Parmi les cires, on peut notamment citer :

- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25 °C, les ozokérites, les esters gras et les glycérides concrets à 25 °C; les cires de polyéthylène et les cires obtenues par synthèse de Fischer-Tropsch; des huiles hydrogénées concrètes à 25 °C; des lanolines; des esters gras concrets à 25 °C; les cires de silicone; les cires fluorées; leurs mélanges.

On peut définir les composés gras pâteux à l'aide d'au moins une des propriétés physico-chimiques suivantes :

- une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Les compositions de l'invention peuvent également comprendre des alkyl, alcoxy ou phényl-diméthicones tels que, par exemple le produit vendu sous la dénomination de "Abil wax 2440" par la Société GOLDSCHMIDT.

Les compositions selon l'invention peuvent également comprendre des résines de silicone comprenant une combinaison des unités $R_3SiO_{1/2}$ , $R_2SiO_{2/2}$ , $RSiO_{3/2}$ et $SiO_{4/2}$, R représentant un groupe alkyle ayant de 1 à 6 atomes de carbone ou un groupe phényle.
De préférence, la partie non volatile de la phase grasse de la composition A est exempte de résine fluorée. De préférence encore, elle est exempte de corps gras fluoré.
La partie non volatile de la phase grasse de la composition A possède des paramètres de solubilité de Hansen $\delta^a_d$, $\delta^a_p$, $\delta^a_h$.
Les paramètres de solubilité sont les paramètres de solubilité de Hansen tels que définis dans l'article de C. M. HANSEN "The three dimensional solubility parameters"", J. paint Technol. 39, 105 (1967) et dans l'ouvrage suivant : "Handbook of solubility Parameters", Allan F. BARTON, CRC Press, 1991.

- $\delta^a_d$ est la composante dispersive du paramètre de solubilité de la phase lipidique de la composition A,
- $\delta^a_p$ est la composante polaire du paramètre de solubilité de la phase lipidique de la composition A,
- $\delta^a_h$ est la composante des liaisons spécifiques (liaisons hydrogène) du paramètre de solubilité de la phase lipidique de la composition A.

Lorsque la partie non volatile de la phase grasse de la composition A est un mélange de corps gras non volatils, les paramètres de solubilité du mélange sont déterminés à partir de ceux des corps gras pris séparément, selon les relations suivantes :

$$\delta_{dmel} = \sum_i xi\, \delta_{di} \quad ; \quad \delta_{pmel} = \sum_i xi\, \delta_{pi} \quad \text{et} \quad \delta_{hmel} = \sum_i xi\, \delta_{hi}$$

où xi représente la fraction volumique du corps gras i dans le mélange, $\delta_{di}$ est la composante dispersive du paramètre de solubilité du corps gras i, $\delta_{pi}$ est la composante polaire du paramètre de solubilité du corps gras i et $\delta_{hi}$ est la composante des liaisons spécifiques (liaisons hydrogène) du paramètre de solubilité du corps gras i.
La phase grasse de la composition A peut éventuellement comprendre une partie volatile. La partie volatile de la phase grasse de la composition A peut ainsi comprendre tout corps gras volatil habituellement utilisé dans les compositions cosmétiques comme par exemple les huiles volatiles.
On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.
Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.

On peut également citer les huiles volatiles hydrocarbonées, telles que les isoparaffines et notamment l'isododécane.
La composition A selon l'invention comprend de préférence une phase particulaire qui peut comprendre des pigments et/ou des nacres et/ou des charges habituellement utilisés dans les compositions cosmétiques.
Par pigments, il faut comprendre des particules blanches ou colorées, minérales ou organiques, insolubles dans le milieu, destinées à colorer et/ou opacifier la composition.
Les pigments peuvent être présents à raison de 0-40 % en poids, par rapport au poids total de la composition, et de préférence à raison de 2-20 %. Ils peuvent être blancs ou colorés, minéraux et/ou organiques, de taille usuelle

ou nanométrique. On peut citer, parmi les pigments et les nanopigments minéraux, les dioxydes de titane, de zirconium ou de cérium, ainsi que les oxydes de zinc, de fer ou de chrome, les nanotitanes, le bleu ferrique. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Les pigments peuvent notamment être enrobés par des composés siliconés tels que des PDMS et/ou par des polymères, notamment des polyéthylènes. On peut ainsi citer les pigments SA de Maprecos ou les pigments PI de Myoshi. En effet, on a constaté que lorsque les pigments n'étaient pas enrobés, le produit obtenu présentait un aspect non lisse et/ou cassant.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges, qui peuvent être présentes dans la composition à raison de 0-40 % en poids, par rapport au poids total de la composition, de préférence 2-20%, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymères d'acrylate telles que l'Expancel (Nobel Industrie), le Polytrap (Dow Corning) et les microbilles de résine de silicone (Tospearls de Toshiba, par exemple).

Par nacres, il faut comprendre des particules irisées qui réfléchissent la lumière.

Les nacres peuvent être présentes dans la composition à raison de 0-40% en poids, de préférence à un taux élevé de l'ordre de 2-20% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

La composition A selon l'invention peut se présenter sous la forme d'une poudre, par exemple compactée, libre ou encore pressée. La phase grasse représente alors de 1 à 30% en poids, par rapport au poids total de la composition, le reste de la composition étant composé de la phase pulvérulente uniquement. Dans le cas d'une poudre libre par exemple, la phase grasse peut représenter de 1 à 5% en poids, par rapport au poids total de la composition. Pour une poudre compactée, la teneur en phase grasse peut représenter de 1 à 20% en poids, par rapport au poids total de la composition.

La composition A peut également comprendre des composés amphiphiles, c'est-à-dire des composés comprenant à la fois une partie lipophile (partie apolaire) et une partie hydrophile (partie polaire) et pouvant s'adsorber à une surface ou une interface. De tels composés sont par exemple les émulsionnants et les coémulsionnants.

Les émulsionnants et les coémulsionnants utilisés dans la composition A lorsqu'elle est sous forme d'émulsion sont choisis parmi ceux classiquement utilisés dans les domaines cosmétique et dermatologique. L'émulsionnant et le coémulsionnant sont présents, dans la composition, en une proportion allant de préférence de 0,3 à 30 % en poids, et mieux de 0,5 à 20 % en poids par rapport au poids total de la composition. La composition A peut, en outre, contenir des vésicules lipidiques.

Comme tensioactif H/E, on peut citer notamment (CTFA) : le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate. Comme tensioactif E/H, on peut citer notamment le mélange polyglycéryl-4 isostéarate/ cétyldiméthicone copolyol/ hexyl laurate et le mélange Mineral oil/petrolatum/ozokérite/glycéryl oléate/alcool de lanoline.

Lorsque la composition A comprend des composés amphiphiles, en particulier lorsqu'elle se présente sous la forme d'une émulsion, les paramètres de solubilité sont calculés comme expliqué ci-dessus dans le cas d'un mélange de corps gras non volatils, en ne tenant compte que de la partie lipophile des composés amphiphiles.

De façon connue, la composition A peut contenir également des adjuvants habituels dans le domaine cosmétique ou dermatologique, tels que les gélifiants hydrophiles ou lipophiles, les additifs hydrophiles ou lipophiles, les conservateurs, les antioxydants, les solvants, les parfums, les filtres solaires, les absorbeurs d'odeur et les matières colorantes. Les quantités de ces différents adjuvants sont celles classiquement utilisées dans ces domaines, et par exemple de 0,01 à 10 % du poids total de la composition. Ces adjuvants, selon leur nature, peuvent être introduits dans la phase grasse, dans la phase aqueuse et/ou dans les vésicules lipidiques.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium.

La composition A peut se présenter sous toutes les formes galéniques à usage topique normalement utilisées et être notamment sous forme de suspension ou dispersion huileuse, d'émulsions obtenues par dispersion d'une phase grasse dans une phase aqueuse (H/E) ou inversement (E/H), d'émulsions triples (E/H/E ou H/E/H) ou de dispersions vésiculaires de type ionique et/ou non ionique, de gel anhydre, de poudres libre, compacte ou préssée. Ces compositions sont préparées selon les méthodes usuelles.

Lorsque la composition est une émulsion, la proportion de la phase grasse peut aller de 5 à 80 % en poids, et de préférence de 5 à 50 % en poids par rapport au poids total de la composition.

Selon une forme préférée de réalisation de l'invention, la composition A se présente sous la forme d'une composition anhydre.

Les compositions A selon l'invention sont de préférence des compositions de maquillage de la peau, des semi-muqueuses, des muqueuses et/ou des phanères, et se présentent alors par exemple sous la forme d'un fond de teint, d'un fard à joues ou à paupières, d'un anticerne, d'un produit de maquillage du corps, d'une poudre, d'un rouge à lèvres, d'un mascara, d'un eye-liner ou d'un vernis à ongles. Elles peuvent se présenter sous toutes les formes classiques de compositions de maquillage telles que les sticks, les bâtons, les pâtes souples, les crèmes, les fluides, les poudres libre, compacte ou pressée.

Selon une forme préférée de réalisation de l'invention, la composition A est une poudre, et de préférence encore une poudre compacte.

La base cosmétique B selon l'invention comprend nécessairement une phase grasse qui comprend au moins une partie non volatile.

La partie non volatile de la phase grasse de la base cosmétique B peut comprendre tous corps gras non volatils déjà cités ci-dessus pour la partie non volatile de la phase grasse de la composition A.

De préférence, les huiles non volatiles sont présentes au sein de la base cosmétique B à une teneur pouvant aller de 1 à 90% , de préférence encore de 10 à 60%, en poids, par rapport au poids total de la composition.

De préférence, la partie non volatile de la base cosmétique B est exempte de résine fluorée. De préférence encore, elle est exempte de corps gras fluoré.

Dans la présente invention, la partie non volatile de la phase grasse de la base cosmétique B a des paramètres de solubilité de Hansen $\delta^b{}_d$, $\delta^b{}_p$, $\delta^b{}_h$. Comme indiqué plus haut, les paramètres de solubilité sont les paramètres de solubilité de Hansen tels que définis dans l'article de C. M. HANSEN "The three dimensional solubility parameters"", J. paint Technol. 39, 105 (1967) et dans l'ouvrage suivant : "Handbook of solubility Parameters", Allan F. BARTON, CRC Press, 1991.

- $\delta^b{}_d$ est la composante dispersive du paramètre de solubilité de la phase lipidique de la base cosmétique B,
- $\delta^b{}_p$ est la composante polaire du paramètre de solubilité de la phase lipidique de la base cosmétique B,
- $\delta^b{}_h$ est la composante des liaisons spécifiques (liaisons hydrogène) du paramètre de solubilité de la phase lipidique de la base cosmétique B.

Comme ci-dessus, lorsque la partie non volatile de la phase grasse de la base cosmétique B est un mélange de corps gras non volatils, les paramètres de solubilité du mélange sont déterminés à partir de ceux des corps gras pris séparément, selon les relations suivantes :

$$\delta_{dmel} = \sum_i xi\, \delta_{di} \quad ; \quad \delta_{pmel} = \sum_i xi\, \delta_{pi} \quad \text{et} \quad \delta_{hmel} = \sum_i xi\, \delta_{hi}$$

où xi représente la fraction volumique du corps gras i dans le mélange.

Dans la présente invention, les paramètres de solubilité de Hansen $\delta^a{}_d$, $\delta^a{}_p$, $\delta^a{}_h$ de la partie non volatile de la phase grasse de la composition A et les paramètres de solubilité de Hansen $\delta^b{}_d$, $\delta^b{}_p$, $\delta^b{}_h$ de la partie non volatile de la phase grasse de la base cosmétique B sont respectivement choisis de telle façon que la distance séparant A de B dans l'espace des paramètres de solubilité de Hansen, à savoir d(A-B), soit supérieure ou égale à 3 $J^{1/2}cm^{-3/2}$, de préférence supérieure ou égale à 5 $J^{1/2}cm^{-3/2}$.

L'éloignement entre les paramètres de solubilité des deux systèmes est calculé à partir de la distance d(A-B) les séparant dans un espace à trois dimensions, chaque dimension correspondant à un paramètre de Hansen selon la relation suivante

$$d(A\text{-}B) = \sqrt{4(\delta^a{}_d - \delta^b{}_d)^2 + (\delta^a{}_p - \delta^b{}_p)^2 + (\delta^a{}_h - \delta^b{}_h)^2}$$

Ainsi, selon la présente invention, $d(A\text{-}B) \geq 3\ J^{1/2}cm^{-3/2}$, et de préférence encore, $d(A\text{-}B) \geq 5\ J^{1/2}cm^{-3/2}$.

La base cosmétique B peut également comprendre des huiles volatiles. Ces huiles volatiles peuvent être choisies parmi celles citées ci-dessus pour la composition A.

La base cosmétique B comprend de préférence un support physiologiquement acceptable, c'est-à-dire compatible avec la peau, les muqueuses y compris les lèvres, et/ou les fibres kératiniques (cheveux, cils).

La base cosmétique B peut également comprendre des composés amphiphiles tels que définis ci-dessus pour la composition A.

Comme tensioactif H/E, on peut citer notamment (CTFA) : le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate. Comme tensioactif E/H, on peut citer notamment le mélange polyglycéryl-4 isostéarate/ cétyldiméthicone copolyol/ hexyl laurate et le mélange Minéral oil/petrolatum/ozokérite/glycéryl oléate/alcool de lanoline.

Elle peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle ou le polyglycéryl 10-décaoléate.

Comme émulsionnants utilisables dans l'invention, on peut citer par exemple le stéarate de glycérol et le PEG-50 stéarate.

Lorsque la base cosmétique B comprend des composés amphiphiles, en particulier lorsqu'elle se présente sous la forme d'une émulsion, les paramètres de solubilité sont calculés comme expliqué ci-dessus dans le cas d'un mélange de corps gras non volatils, en ne tenant compte que de la partie lipophile des composés amphiphiles.

Lorsque la base cosmétique B est sous la forme d'une émulsion, elle comprend une phase aqueuse qui peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

Les bases cosmétiques B de l'invention contiennent en outre un milieu cosmétiquement, hygiéniquement, pharmaceutiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

De préférence, la base cosmétique B est une composition de soin de la peau et/ou des muqueuses et/ou des semi-muqueuses.

La base cosmétique B selon l'invention peut contenir d'autres composés classiquement utilisés dans les compositions de soin et destinés notamment à la prévention et/ou au traitement des affections cutanées.

Elle peut ainsi comprendre des antioxydants, des parfums, des huiles essentielles, des conservateurs, des actifs cosmétiques ou pharmaceutiques lipophiles ou hydrophiles, des hydratants, des vitamines, des acides gras essentiels, des sphingolipides, des composés auto-bronzants tels que la DHA, des filtres solaires.

Comme autres composés, on peut citer par exemple les polyols tels que la glycérine, les glycols et les dérivés de sucre, les enzymes, les vitamines telles que la vitamine C (acide ascorbique), la vitamine A (rétinol), la vitamine D, la vitamine E (tocophérol), la vitamine K et les dérivés de ces vitamines tels que les esters, les céramides, les dépigmentants tels que l'acide kojique et l'acide caféique, les bêtahydroxyacides tels que l'acide salicylique et ses dérivés, les alpha-hydroxyacides tels que l'acide lactique et l'acide glycolique, les hydratants tels que les hydrolysats de protéines, les adoucissants tels que l'allantoïne et leurs mélanges.

La base cosmétique B peut se présenter sous toutes les formes galéniques à usage topique normalement utilisées et être notamment sous forme de solution huileuse, de suspension aqueuse ou huileuse ou de dispersion, d'émulsions obtenues par dispersion d'une phase grasse dans une phase aqueuse (H/E) ou inversement (E/H), d'émulsions triples (E/H/E ou H/E/H) ou de dispersions vésiculaires de type ionique et/ou non ionique, de gel anhydre. Ces compositions sont préparées selon les méthodes usuelles.

Lorsque la composition est une émulsion, la proportion de la phase grasse peut aller de 5 à 80 % en poids, et de préférence de 5 à 50 % en poids par rapport au poids total de la composition.

Selon une forme préférée de réalisation de l'invention, la base cosmétique B se présente sous la forme d'une émulsion, et de préférence encore sous la forme d'une émulsion H/E.

Bien entendu, pour la base cosmétique B comme pour la composition A, l'homme du métier veillera à choisir ce ou ces éventuels composés complémentaires, et/ou leur quantité, de manière telle que les propriétés avantageuses du procédé selon l'invention ne soient pas, ou substantiellement pas, altérées par l'adjonction envisagée.

Selon le procédé de la présente invention, on applique, dans une première étape (étape 1), la base cosmétique B définie ci-dessus sur la peau, les muqueuses, les semi-muqueuses ou les phanères. Dans une deuxième étape (étape 2), on applique la composition A définie ci-dessus sur la base cosmétique B.

De préférence, lorsque la base cosmétique B comprend des composés volatils tels que par exemple les huiles volatiles décrites ci-dessus, le procédé ci-dessus comprend une étape supplémentaire de séchage entre l'étape 1 et l'étape 2. Cette étape de séchage peut s'effectuer à l'air ambiant ou à l'aide d'air chaud. Elle peut par exemple avoir une durée de 10 minutes environ.

Le produit de maquillage obtenu sur la peau par ce procédé présente des propriétés sans transfert remaquables.

L'invention est illustrée plus en détails dans les exemples suivants.
Dans les exemples suivants, les quantités sont données en pourcentage en poids par rapport au poids total de la composition.

**Exemple 1 :**

La Demanderesse a réalisé la base cosmétique B suivante sous la forme d'une émulsion H/E :

Composition B :

- beurre de karité 10 %
- huile minérale 3,4 %
- gomme de xanthane 0,1 %
- cyclométhicone 6 %
- eau 67 %
- glycérine 5 %
- stéarate de glycérol / PEG-100 stéarate 2,4 %
- polysorbate 60 1 %
- alcool cétylique 0,6 %
- acide stéarique 0,9 %
- PEG-2 stéarate 1,1 %
- linoléate de glycérol / oléate de glycérol / linolénate de glycérol 2,2 %
- conservateur qsp 100 %

Cette émulsion a été réalisée selon les méthodes classique de préparation des émulsions H/E.
Les valeurs des paramètres de solubilité ont été calculées comme expliqué dans la description de la présente demande en prenant en compte les corps gras non volatils et les parties lipophiles des composés amphiphiles, à savoir : l'alcool cétylique, l'acide stéarique, la fraction liquide du beurre de karité (assimilée à un triglycéride d'acide oléique), l'huile minérale (assimilée à un mélange 30/70 d'hydrocarbures naphtaléniques et paraffiniques de longueur moyenne $C_{26}$ et $C_{24}$, les parties lipophiles du monostéarate de glycérol, du PEG-2 stéarate, du mélange linoléate de glycérol, oléate de glycérol, linolenate de glycérol (mélange assimilé au linoléate de glycérol). Les parties lipophiles du PEG-100 stéarate et du polysorbate 60 ont été considérés comme négligeables.
Les valeurs obtenues sont les suivantes :

- $\delta_d \cong 16{,}3\ J^{1/2}.cm^{-3/2}$
- $\delta_p \cong 1{,}0\ J^{1/2}.cm^{-3/2}$
- $\delta_h \cong 4{,}0\ J^{1/2}.cm^{-3/2}$

La Demanderesse a ensuite réalisé les compositions A suivantes. Ces compostions sont des poudres, pour le visage par exemple.

Composition A1 :

- acétylcitrate de tributyle 8 %
- oxyde de fer (rouge) 5 %
- talc 87 %

Les paramètres de solubilité de la partie non volatile de la composition A1 sont ceux de l'acétyl citrate de tributyle, à savoir:

- $\delta_d = 17.2\ J^{1/2}\ cm^{-3/2}$
- $\delta_p = 16.7\ J^{1/2}\ cm^{-3/2}$

- $\delta_h = 3.0\ J^{1/2}\ cm^{-3/2}$

Ainsi, la distance d(A1-B) est :

$$d(A1\text{-}B) = 15.83\ J^{1/2}\ cm^{-3/2}$$

On applique, par exemple sur le visage d'une personne, la composition B. Puis on applique ensuite la composition A1. Cette dernière transfère très peu.

Composition A2 : comparatif

- cetyl dimethicone, dimethicone et triméthylsiloxysilicate 8 %
- oxyde de fer (rouge) 5 %
- talc 87 %

Les paramètres de solubilité de la partie non volatile de la composition A2 sont ceux de l'huile , à savoir.:

- $\delta_d \cong 17\ J^{1/2}.cm^{-3/2}$
- $\delta_p \cong 1{,}6\ J^{1/2}.cm^{-3/2}$
- $\delta_h \cong 2{,}4\ J^{1/2}.cm^{-3/2}$

Ainsi, la distance d(A2-B) est : 1,85 $J^{1/2}\ cm^{-3/2}$

On applique les compositions B et A2 comme indiqué ci-dessus. La composition A2 transfère beaucoup.

Composition A3 :

- N-neopentanoyl-2-octyl dodecylamine 8 %
- oxyde de fer (rouge) 5 %
- talc 87 %

La distance calculée d(A3-B) est :

$$d(A3\text{-}B) = 6{,}16\ J^{1/2}\ cm^{-3/2}$$

On applique, par exemple sur le visage d'une personne, la composition B. Puis on applique ensuite la composition A3. Cette dernière transfère très peu.

## Revendications

1. Procédé pour améliorer les propriétés 'sans transfert' et la tenue d'une composition A comprenant au moins une phase grasse dont la partie non volatile a des paramètres de solubilité de Hansen $\delta^a_d$, $\delta^a_p$, $\delta^a_h$, comprenant les étapes suivantes :

   - 1°) dans une première étape, on applique sur la peau et/ou les muqueuses et/ou les semi-muqueuses une base cosmétique B comprenant au moins une phase grasse dont la partie non volatile a des paramètres de solubilité de Hansen $\delta^b_d$, $\delta^b_p$, $\delta^b_h$, B étant choisie de telle façon que la distance d(A-B) séparant A de B dans l'espace des paramètres de solubilité de Hansen vérifie la relation suivante :

$$d(A\text{-}B) \geq 3\ J^{1/2}cm^{-3/2},$$

dans laquelle :

$$d(A\text{-}B) = \sqrt{4(\delta^a_d - \delta^b_d)^2 + (\delta^a_p - \delta^b_p)^2 + (\delta^a_h - \delta^b_h)^2}$$

- 2°) dans une deuxième étape, on applique sur la base B la composition A.

**2.** Procédé de maquillage, en particulier de la peau et/ou des muqueuses et/ou des semi-muqueuses, comprenant les étapes suivantes :

- 1°) dans une première étape, on applique sur la peau et/ou les muqueuses et/ou les semi-muqueuses une base cosmétique B comprenant au moins une phase grasse dont la partie non volatile a des paramètres de solubilité de Hansen $\delta^b_d$, $\delta^b_p$, $\delta^b_h$,

- 2°) dans une deuxième étape, on applique sur la base B une composition A comprenant au moins une phase grasse dont la partie non volatile a des paramètres de solubilité de Hansen $\delta^a_d$, $\delta^a_p$, $\delta^a_h$,

A et B étant choisies de telle façon que la distance d(A-B) séparant A de B dans l'espace des paramètres de solubilité de Hansen vérifie la relation suivante :

$$d(A\text{-}B) \geq 3\ J^{1/2}cm^{-3/2},$$

dans laquelle :

$$d(A\text{-}B) = \sqrt{4(\delta^a_d - \delta^b_d)^2 + (\delta^a_p - \delta^b_p)^2 + (\delta^a_h - \delta^b_h)^2}$$

**3.** Procédé selon la revendication 1 ou 2, caractérisé par le fait que d(A-B) ≥ 5 $J^{1/2}cm^{-3/2}$.

**4.** Procédé selon l'une quelconque des revendications précédentes, caractérisé par le fait que la partie non volatile de la phase grasse de la composition A comprend une ou plusieurs huiles choisies parmi l'huile de sésame, la lanoline, le myristate d'isopropyle, l'huile de mygliol, le néopentanoate d'isostéaryle, le palmitate d'éthyl-2-hexyle, l'huile de ricin, l'acétyl-citrate de tri-butyle, l'octyl-2-dodecanol, le N-pentanoyl-2-octyl dodecylamine.

**5.** Procédé selon la revendication précédente, caractérisé par le fait que les huiles non volatiles sont présentes dans la composition A à une teneur pouvant aller de 1 à 85%, de préférence encore de 5 à 50%, en poids, par rapport au poids total de la composition.

**6.** Procédé selon l'une quelconque des revendications précédentes, caractérisé par le fait que la phase grasse de la composition A comprend en outre une partie volatile.

**7.** Procédé selon l'une quelconque des revendications précédentes, caractérisé par le fait que la composition A comprend une phase particulaire comprenant des pigments et/ou des nacres et/ou des charges.

**8.** Procédé selon la revendication précédente, caractérisé par le fait que les pigments sont choisis parmi les dioxydes de titane, de zirconium ou de cérium, les oxydes de zinc, de fer ou de chrome, les nanotitanes, le bleu ferrique, le noir de carbone, les laques telles que des sels de calcium, de baryum, d'aluminium ou de zirconium, de colorants acides tels que les colorants halogéno-acides, azoïques ou anthraquinoniques.

**9.** Procédé selon la revendication 7 ou 8, caractérisé par le fait que les pigments sont présents dans la composition A à une teneur pouvant aller jusqu'à 40% en poids, par rapport au poids total de la composition.

**10.** Procédé selon l'une quelconque des revendications 7 à 9, caractérisé par le fait que les charges sont choisies parmi le talc, le mica, la silice, le kaolin, les poudres de Nylon et de polyéthylène, le Téflon, l'amidon, le nitrure de

bore, les microsphères de polymères d'acrylate et les microbilles de résine de silicone.

**11.** Procédé selon l'une quelconque des revendications 7 à 10, caractérisé par le fait que les charges peuvent être présentes dans la composition A à une teneur allant jusqu'à 40% en poids, par rapport au poids total de la composition.

**12.** Procédé selon l'une quelconque des revendications 7 à 11, caractérisé par le fait que les nacres sont choisies parmi la nacre naturelle, le mica recouvert d'oxyde de titane, d'oxyde de fer, de pigment naturel ou d'oxychlorure de bismuth, le mica titane coloré.

**13.** Procédé selon l'une quelconque des revendications 7 à 12, caractérisé par le fait que les nacres peuvent être présentes dans la composition A à une teneur allant jusqu'à 40% en poids, par rapport au poids total de la composition.

**14.** Procédé selon l'une quelconque des revendications précédentes, caractérisé par le fait que la composition A est sous forme de suspension ou dispersion huileuse, d'émulsions obtenues par dispersion d'une phase grasse dans une phase aqueuse (H/E) ou inversement (E/H), d'émulsions triples (E/H/E ou H/E/H) ou de dispersions vésiculaires de type ionique et/ou non ionique, de gel anhydre, de poudres libre, compacte ou pressée.

**15.** Procédé selon l'une quelconque des revendications précédentes, caractérisé par le fait que la composition A est une composition anhydre.

**16.** Procédé selon l'une quelconque des revendications précédentes, caractérisé par le fait que la composition A se présente sous la forme d'un fond de teint, d'un fard à joues ou à paupières, d'un anticerne, d'un produit de maquillage du corps, d'une poudre, d'un rouge à lèvres, d'un mascara, d'un eye-liner ou d'un vernis à ongles.

**17.** Procédé selon l'une quelconque des revendications précédentes, caractérisé par le fait que la composition A est une poudre compacte.

**18.** Procédé selon l'une quelconque des revendications précédentes, caractérisé par le fait que la partie non volatile de la phase grasse de la base cosmétique B comprend des huiles non volatiles.

**19.** Procédé selon la revendication précédente, caractérisé par le fait que les huiles non volatiles sont présentes au sein de la base cosmétique B à une teneur pouvant aller de 1 à 90% , de préférence encore de 10 à 60%, en poids, par rapport au poids total de la composition.

**20.** Procédé selon l'une quelconque des revendications précédentes, caractérisé par le fait que la base cosmétique B comprend de composés volatils.

**21.** Procédé selon la revendication précédente, caractérisé par le fait qu'il comprend une étape supplémentaire de séchage entre la première étape et la deuxième étape.

**22.** Procédé selon l'une quelconque des revendications précédentes, caractérisé par le fait que la base cosmétique B comprend au moins un composé amphiphile choisi parmi le cétéaryl-glucoside, le PEG-40 stéarate, le sorbitan tristéarate, le sorbitan stéarate, le polysorbate 60, le mélange sorbitan stéarate/sucrose co-coate, le mélange de glycéryl stéarate/PEG-100 stéarate, le PEG-400, le stéarate de glycéryle, le mélange de PEG-6/PEG-32/glycol stéarate, le mélange polyglycéryl-4 isostéarate/ cétyldiméthicone copolyol/ hexyl laurate et/ou le mélange Minéral oil/petrolatum/ozokérite/glycéryl oléate/alcool de lanoline.

**23.** Procédé selon l'une quelconque des revendications précédentes, caractérisé par le fait que la base cosmétique B comprend au moins un composé choisi parmi les antioxydants, les parfums, les huiles essentielles, les conservateurs, les actifs cosmétiques ou pharmaceutiques lipophiles ou hydrophiles, les acides gras essentiels, les sphingolipides, les composés auto-bronzants tels que la DHA, les filtres solaires, les polyols tels que la glycérine, les glycols et les dérivés de sucre, les enzymes, les vitamines telles que la vitamine C, la vitamine A, la vitamine D, la vitamine E, la vitamine K et les dérivés de ces vitamines tels que les esters, les céramides, les dépigmentants tels que l'acide kojique et l'acide caféique, les bêtahydroxyacides tels que l'acide salicylique et ses dérivés, les alpha-hydroxyacides tels que l'acide lactique et l'acide glycolique, les hydratants tels que les hydrolysats de protéines, les adoucissants tels que l'allantoïne et leurs mélanges.

**24.** Procédé selon l'une quelconque des revendications précédentes, caractérisé par le fait que la base cosmétique B se présente sous la forme de solution huileuse, de suspension aqueuse ou huileuse ou de dispersion, d'émulsions obtenues par dispersion d'une phase grasse dans une phase aqueuse (H/E) ou inversement (E/H), d'émulsions triples (E/H/E ou H/E/H) ou de dispersions vésiculaires de type ionique et/ou non ionique, de gel anhydre.

**25.** Procédé selon la revendication précédente, caractérisé par le fait que la base cosmétique B est une émulsion H/E.

**26.** Procédé selon l'une quelconque des revendications précédentes, caractérisé par le fait que la base cosmétique B est une composition de soin de la peau et/ou des muqueuses et/ou des semi-muqueuses.

**27.** Composition A, en particulier de maquillage, comprenant une phase grasse, caractérisée par le fait que les paramètres de solubilité de Hansen $\delta^a{}_d$, $\delta^a{}_p$, $\delta^a{}_h$ de la partie non volatile de ladite phase grasse sont choisis de telle façon que la distance d(A-B) séparant, dans l'espace des paramètres de solubilité de Hansen, A d'une composition B telle que définie à l'une quelconque des revendications 1, 2 et 18 à 26 vérifie la relation suivante :

$$d(A\text{-}B) \geq 3\ J^{1/2}cm^{-3/2},$$

dans laquelle :

$$d(A\text{-}B) = \sqrt{4(\delta^a{}_d - \delta^b{}_d)^2 + (\delta^a{}_p - \delta^b{}_p)^2 + (\delta^a{}_h - \delta^b{}_h)^2}$$

et où $\delta^b{}_d$, $\delta^b{}_p$, $\delta^b{}_h$ sont les paramètres de solubilité de Hansen de la partie non volatile de la phase grasse de B.

**28.** Base cosmétique B, en particulier de soin, comprenant une phase grasse, caractérisée par le fait que les paramètres de solubilité de Hansen $\delta^b{}_d$, $\delta^b{}_p$, $\delta^b{}_h$ de la partie non volatile de ladite phase grasse sont choisis de telle façon que la distance d(A-B) séparant, dans l'espace des paramètres de solubilité de Hansen, B d'une composition A telle que définie à l'une quelconque des revendications 1 à 17 vérifie la relation suivante :

$$d(A\text{-}B) \geq 3\ J^{1/2}cm^{-3/2},$$

dans laquelle :

$$d(A\text{-}B) = \sqrt{4(\delta^a{}_d - \delta^b{}_d)^2 + (\delta^a{}_p - \delta^b{}_p)^2 + (\delta^a{}_h - \delta^b{}_h)^2}$$

et où $\delta^a{}_d$, $\delta^a{}_p$, $\delta^a{}_h$ sont les paramètres de solubilité de Hansen de la partie non volatile de la phase grasse de A.

**29.** Utilisation d'une composition A telle que définie à la revendication 27 pour la mise en oeuvre d'un procédé tel que défini à l'une quelconque des revendications 1 à 26.

**30.** Utilisation d'une base cosmétique B telle que définie à la revendication 28 pour la mise en oeuvre d'un procédé tel que défini à l'une quelconque des revendications 1 à 26.

**31.** Kit comprenant au moins une composition A telle que définie à la revendication 27 et au moins une base cosmétique B telle que définie à la revendication 28.

**32.** Produit de maquillage sans transfert bicouche constitué d'une première couche, en contact avec la peau et/ou les muqueuses et/ou les semi-muqueuses, constituée de la base cosmétique B telle que définie à la revendication 28 et d'une deuxième couche, constituée de la composition A telle que définie à la revendication 27, apposée sur la première couche.

**Office européen des brevets**

## RAPPORT PARTIEL DE RECHERCHE EUROPEENNE

qui selon la règle 45 de la Convention sur le brevet européen est consideré, aux fins de la procédure ultérieure, comme le rapport de la recherche européenne

Numéro de la demande
EP 99 40 2437

### DOCUMENTS CONSIDERES COMME PERTINENTS

| | Catégorie | Citation du document avec indication, en cas de besoin, des parties pertinentes | Revendication concernée | CLASSEMENT DE LA DEMANDE (Int.Cl.7) |
|---|---|---|---|---|
| 5 | A | WO 98 16196 A (PROCTER & GAMBLE) 23 avril 1998 (1998-04-23) * le document en entier * --- | 4-26 | A61K7/02 A61K7/027 |
| 2 | A | PATENT ABSTRACTS OF JAPAN vol. 010, no. 172 (C-354), 18 juin 1986 (1986-06-18) & JP 61 024512 A (KOBAYASHI KOOSEE:KK), 3 février 1986 (1986-02-03) * abrégé * --- | 4-16 | |
| 3 | A | DATABASE WPI Section Ch, Week 9821 Derwent Publications Ltd., London, GB; Class A26, AN 98-234675 XP002108431 & JP 10 072315 A (SHISEIDO CO LTD), 17 mars 1998 (1998-03-17) * abrégé * --- | 4-16 | |
| 5 | A | US 5 238 678 A (NAKAMURA NAOKI ET AL) 24 août 1993 (1993-08-24) * revendications * --- -/-- | 4-26 | DOMAINES TECHNIQUES RECHERCHES (Int.Cl.7) A61K |

### RECHERCHE INCOMPLETE

**La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.**

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| BERLIN | 20 décembre 1999 | Pelli Wablat, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen
des brevets

**RECHERCHE INCOMPLETE**
**FEUILLE SUPPLEMENTAIRE C**

Numéro de la demande
EP 99 40 2437

Revendications ayant fait
l'objet de recherches incomplètes:
4-26

Revendications n'ayant pas fait
l'objet de recherches:
1-3 27-32

Raison pour la limitation de la recherche:

Les revendications 1-3 27-32 sont basées sur des paramètres physiques.

L'utilisation de ces paramètres est considérée , dans le présent contexte, comme menant à un manque de clarté au sens de l'Article 84 CBE. Il est impossible de comparer les paramètres que le déposant a choisi d'utiliser avec ce qui est révélé dans l'état de la technique. Le manque de clarté qui en découle est tel q'une recherche significative complète est impossible. Par conséquent, une recherche incomplete a été effectuée pour les revendications 4-26. Les revendications 1-3 et 27-32 n'ont pas eté recherchées.

Office européen des brevets

# RAPPORT PARTIEL DE RECHERCHE EUROPEENNE

Numero de la demande
EP 99 40 2437

| | DOCUMENTS CONSIDERES COMME PERTINENTS | | | CLASSEMENT DE LA DEMANDE (Int.Cl.7) |
|---|---|---|---|---|
| | Catégorie | Citation du document avec indication, en cas de besoin, des parties pertinentes | Revendication concernée | |
| 5 | A | EP 0 847 752 A (OREAL)<br>17 juin 1998 (1998-06-17)<br>* revendications *<br>--- | 4-24 | |
| 5 | A | WO 97 17057 A (PROCTER & GAMBLE)<br>15 mai 1997 (1997-05-15)<br>* le document en entier *<br>----- | 4-26 | |
| | | | | DOMAINES TECHNIQUES RECHERCHES (Int.Cl.7) |

EPO FORM 1503 03.82 (P04C11)

## ANNEXE AU RAPPORT DE RECHERCHE EUROPEENNE RELATIF A LA DEMANDE DE BREVET EUROPEEN NO. EP 99 40 2437

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-12-1999

| Document brevet cité au rapport de recherche | Date de publication | Membre(s) de la famille de brevet(s) | Date de publication |
|---|---|---|---|
| WO 9816196 A | 23-04-1998 | AU 5149098 A | 11-05-1998 |
| | | CZ 9901347 A | 15-09-1999 |
| | | EP 0930871 A | 28-07-1999 |
| JP 61024512 A | 03-02-1986 | AUCUN | |
| JP 10072315 A | 17-03-1998 | AUCUN | |
| US 5238678 A | 24-08-1993 | JP 5025021 A | 02-02-1993 |
| | | JP 2955072 B | 04-10-1999 |
| | | JP 5058840 A | 09-03-1993 |
| | | FR 2679769 A | 05-02-1993 |
| EP 0847752 A | 17-06-1998 | FR 2756176 A | 29-05-1998 |
| | | BR 9705926 A | 27-04-1999 |
| | | CA 2220033 A | 26-05-1998 |
| | | JP 10158118 A | 16-06-1998 |
| WO 9717057 A | 15-05-1997 | AU 7476096 A | 29-05-1997 |
| | | AU 7476196 A | 29-05-1997 |
| | | AU 7522096 A | 29-05-1997 |
| | | BR 9611420 A | 23-02-1999 |
| | | BR 9611421 A | 23-02-1999 |
| | | BR 9611431 A | 23-02-1999 |
| | | CA 2236790 A | 15-05-1997 |
| | | CA 2236942 A | 15-05-1997 |
| | | CA 2236974 A | 15-05-1997 |
| | | CZ 9801404 A | 14-10-1998 |
| | | CZ 9801406 A | 14-10-1998 |
| | | CZ 9801408 A | 16-09-1998 |
| | | EP 0862411 A | 09-09-1998 |
| | | EP 0862412 A | 09-09-1998 |
| | | EP 0868169 A | 07-10-1998 |
| | | HU 9900113 A | 28-05-1999 |
| | | HU 9901986 A | 29-11-1999 |
| | | NO 982033 A | 07-07-1998 |
| | | NO 982034 A | 07-07-1998 |
| | | NO 982035 A | 07-07-1998 |
| | | NZ 321430 A | 28-10-1999 |
| | | NZ 321800 A | 28-10-1999 |
| | | PL 326676 A | 12-10-1998 |
| | | PL 326679 A | 12-10-1998 |
| | | PL 326705 A | 26-10-1998 |
| | | SK 59298 A | 04-11-1998 |
| | | SK 59398 A | 04-11-1998 |
| | | SK 59498 A | 04-11-1998 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO. EP 99 40 2437

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-12-1999

| Document brevet cité au rapport de recherche | Date de publication | Membre(s) de la famille de brevet(s) | Date de publication |
|---|---|---|---|
| WO 9717057 A | | WO 9717058 A | 15-05-1997 |
| | | WO 9717059 A | 15-05-1997 |

EPO FORM P0460

Pour tout renseignement concernant cette annexe : voir Journal Officiel de l'Office européen des brevets, No.12/82